# EUROPÄISCHE PATENTSCHRIFT

(11) **EP 2 787 057 B1**
(45) Veröffentlichungstag und Bekanntmachung des Hinweises auf die Patenterteilung: **26.02.2020**
(21) Anmeldenummer: 14161868.6
(22) Anmeldetag: 27.03.2014
(51) Int. Cl.: C09K 11/88, B01J 13/00, B01J 13/02, B01J 13/22, B82Y 20/00, B82Y 40/00, G01N 33/58, H01L 21/02, H01L 31/0352, H01L 33/00, H01L 33/28, H01L 33/44

(54) **Blau-emittierende Leuchtdioden auf Basis von Zinkselenid-Quantenpunkten**
Blue-emitting lightdiodes based on zinc-selenide quantum dots
Diode électroluminescente émettant dans le bleu à base de points quantiques de séléniure de zinc

(30) Priorität: 05.04.2013 DE 102013206077
(43) Veröffentlichungstag der Anmeldung: 08.10.2014
(73) Patentinhaber: Fraunhofer-Gesellschaft zur Förderung der angewandten Forschung e.V., 80686 München (DE); Korea Electronics Technology Institute, Gyeonggi-do 463-816 (KR)
(72) Erfinder: Greco, Tonino, 10119 Berlin (DE); Ippen, Christian, 14471 Potsdam (DE); Wedel, Armin, 14513 Teltow (DE)
(74) Vertreter: Lux, Berthold

(56) Entgegenhaltungen:
- EP-A1- 1 787 659
- WO-A2-2006/116337
- WO-A2-2007/057182
- WO-A2-2009/025913
- GB-A- 2 470 131
- CUMBERLAND S L ET AL: "INORGANIC CLUSTERS AS SINGLE-SOURCE PRECURSORS FOR PREPARATION OF CDSE, ZNSE, AND CDSE/ZNS NANOMATERIALS", CHEMISTRY OF MATERIALS, AMERICAN CHEMICAL SOCIETY, US, Bd. 14, Nr. 4, 1. April 2002 (2002-04-01), Seiten 1576-1584, XP001163186, ISSN: 0897-4756, DOI: 10.1021/CM010709K
- L S LI, N PRADHAN, Y WANG, X PENG: "High quality ZnSe and ZnS Nanocrystals Formed by Activating Zinc Carboxylate Precursors", NANOLETTERS, Bd. 4, Nr. 11, 10. Oktober 2004 (2004-10-10), Seiten 2261-2264, XP002723839,

## Beschreibung

Die vorliegende Erfindung betrifft ein Verfahren zur Herstellung von kolloidalen, lösungsprozessierbaren Zinkselenid-Quantenpunkten.

Kolloidale Halbleiter-Nanopartikel, auch Quantenpunkte genannt, sind aufgrund ihrer optischen Eigenschaften insbesondere von Interesse für die Anwendung in optoelektronischen Bauelementen. Zu den vorteilhaften Eigenschaften solcher kolloidalen Quantenpunkte zählen u.a. gute Lumineszenzeffizienzien, Einstellbarkeit der Emissionswellenlänge über die Partikelgröße sowie insbesondere die geringe Breite des Emissionspeaks, die in einer hohen Farbsättigung resultiert. Darüber hinaus sind kolloidale Quantenpunkte in organischen Lösungsmitteln löslich, was den Einsatz von günstigen und schnellen lösungsbasierten Prozessierungsmethoden wie zum Beispiel Druckverfahren ermöglicht.

Insbesondere die Anwendung der Quantenpunkte als Emittermaterial in Leuchtdioden ist aus verschiedenen Gründen von hohem Interesse. Durch die hohe Lumineszenzeffizienz der Quantenpunkte können gute Lichtausbeuten erreicht werden und somit der Energieverbrauch für Displays und Beleuchtung gesenkt werden. Durch die schmalen Emissionspeaks wird eine außerordentlich gute Farbsättigung erreicht, wodurch der Farbraum von vollfarbigen Displays erweitert werden kann. Die typischerweise sehr schnelle und einfache Herstellung von Quantenpunkten in einer Ein-Topf-Synthese stellt einen Kostenvorteil gegenüber anderen Emittermaterialien wie metallorganischen Komplexen dar, die in aufwändigen mehrstufigen Syntheseverfahren mit mehreren damit verbundenen Reinigungsschritten erhalten werden. Die Löslichkeit der Quantenpunkte in organischen Lösungsmitteln erlaubt die kostengünstige und flexible Verarbeitung mittels Druckprozessen.

Häufig werden Quantenpunkte aus II-VI-Halbleitern, vornehmlich auf Basis von Cadmiumverbindungen wie CdSe, CdS oder CdTe synthetisiert. Cadmiumverbindungen weisen jedoch eine hohe Toxizität auf. Darüber hinaus bestehen entsprechende umweltpolitische Regulierungsvorschriften (z.B. die RoHS-Direktive in der Europäischen Union), welche die Verwendung von cadmiumhaltigen Materialien einschränken oder komplett verbieten, wodurch die Aussichten für den realen Einsatz dieser Quantenpunkte in beispielsweise optoelektronischen Bauelementen gering sind. Daher besteht ein Bedarf an Quantenpunkten, die sowohl cadmiumfrei sind als auch eine ausreichende Lumineszenzquantenausbeute aufweisen. Ferner sollen diese Quantenpunkte auf eine einfache Weise herstellbar sein.

Als Alternative zu den erwähnten cadmiumhaltigen Materialien für Quantenpunkte werden häufig III-V-Halbleiter vorgeschlagen, beispielsweise Indiumphosphid oder Indiumarsenid. Auch wenn in der Entwicklung dieser Quantenpunkt-Materialien in den vergangenen Jahren weitreichende Fortschritte erzielt wurden, so sind diese Materialien für die Emission blauen Lichts doch ungeeignet, da die intrinsische Bandlücke des entsprechenden Bulkmaterials zu klein ist. Hypothetische blau emittierende Quantenpunkte dieser Materialien müssten daher eine sehr kleine Partikelgröße aufweisen, wodurch dieser aufgrund der hohen Oberflächenenergie thermodynamisch instabil werden. Typischerweise kann mit InP-Quantenpunkten der Bereich von blaugrünem bis nahinfrarotem Licht abgedeckt werden, InAs-Quantenpunkte emittieren im Infrarot-Bereich.

Weitere Alternativen zu cadmiumhaltigen Quantenpunkten sind ternäre oder quaternäre Chalcopyrit-Verbindungen wie Kupferindiumsulfid oder Kupferindiumgalliumsulfid, die allerdings auch nur den Bereich von gelbem bis nahinfrarotem Licht abdecken und darüber hinaus im Allgemeinen aufgrund von stöchiometrischer Inhomogenität einen sehr breiten Emissionspeak aufweisen. Metalldotierte Zinkchalcogenide sind weitere interessante Alternativen, allerdings ist die Einstellbarkeit der Emissionsfarbe über die Partikelgröße eingeschränkt, vielmehr ist das Dotierungsmaterial für die Emissionsfarbe entscheidend. Durch Mangan-Dotierung von Zinksulfid-Nanopartikeln sind gelb oder orange emittierende Materialien zugänglich, Cu-Dotierung erlaubt auch grün emittierende Materialien. WO 2009/025913 beschreibt Kern/Schale Nanokristalle und ein Verfahren zur Herstellung der Kristalle.

Es besteht also nach wie vor der Bedarf für ein cadmiumfreies Quantenpunktsystem, das mit hoher Effizienz und Farbsättigung tiefblaues Licht emittiert, einfach herzustellen ist und durch lösungsbasierte Prozesse verarbeitet werden kann, und beispielsweise in einer Dünnschicht-Leuchtdiode verwendet werden kann.

Demnach ist es eine Aufgabe der vorliegenden Erfindung, cadmiumfreie Quantenpunkte bereitzustellen, welche die Vorteile bekannter cadmiumhaltiger Quantenpunkte aufweisen. Es ist insbesondere Aufgabe der vorliegenden Erfindung, Quantenpunkte bereitzustellen, die sowohl cadmiumfrei sind als auch eine gute Lumineszenzquantenausbeute aufweisen. Eine weitere Aufgabe besteht darin, eine Synthese für die besagten Quantenpunkte bereitzustellen, die eine Kontrolle der Partikelgröße und der Emissionswellenlänge ermöglicht. Eine zusätzliche Aufgabe besteht darin, dass die Quantenpunkte durch ein einfaches Verfahren bereit gestellt werden können. Insbesondere besteht eine Aufgabe darin, dass das Verfahren ohne Reinigungsprozesse nach den jeweiligen Zwischenschritten durchgeführt werden kann.

Die Lösung der genannten und weiterer Aufgaben ergibt sich aus den Gegenständen der unabhängigen Ansprüche. Vorteilhafte Ausführungsformen ergeben sich durch deren Kombination mit den Merkmalen der abhängigen Ansprüche.

Gemäß einem Aspekt der vorliegenden Erfindung wird ein Verfahren gemäß Anspruch 1 zur Herstellung kolloidaler Quantenpunkte bereitgestellt, umfassend
a) einen Kern umfassend Zinkselenid (ZnSe)
b) optional eine oder mehrere auf den Kern aufgewachsene Schalen umfassend Zinkselenid
c) mindestens eine auf den Kern und/oder die letzte Schale aufgetragene Schale aus einem passivierenden Material,
wobei die kolloidalen Quantenpunkte cadmiumfrei sind.

Im Rahmen der vorliegenden Erfindung wird der Begriff "kolloidal" in seiner üblichen, dem Fachmann geläufigen Bedeutung verstanden und bezieht sich auf eine Dispersion von Teilchen in einem anderen festen, flüssigen oder gasförmigen Medium.

Im Rahmen der vorliegenden Erfindung wird der Begriff "Quantenpunkt" in seiner üblichen, dem Fachmann geläufigen Bedeutung verstanden und bezieht sich auf einen Kern aus kolloidalen Halbleiter-Nanopartikeln, auf welchen mehrere Schalen epitaktisch aufgeschichtet werden.

Im Rahmen der vorliegenden Erfindung bezieht sich der Begriff "Kern" auf Nanopartikel, welche Halbleiter-Materialien umfassen. Vorzugsweise bestehen die Kerne aus Zinkselenid.

Im Rahmen der vorliegenden Erfindung wird der Begriff "Nanopartikel" in seiner üblichen, dem Fachmann geläufigen Bedeutung verstanden und bezieht sich auf Partikel, die aus einem Verbund von Atomen oder Molekülen bestehen und deren Größe zwischen 1 und 100 nm liegt.

Im Rahmen der vorliegenden Erfindung bezieht sich der Begriff "Schalen" auf Schichten umfassend Halbleiter-Materialien oder passivierende Materialien, welche den Kern umgeben. Vorzugsweise bestehen die Schalen umfassend Halbleiter-Materialien aus Zinkselenid und den Schalen umfassend passivierende Materialien aus Zinksulfid.

Im Rahmen der vorliegenden Erfindung bezieht sich der Begriff "epitaktisch" auf das Aufbringen der einzelnen Schalen auf das Substrat, wobei die erste Schale auf den Kern aufgewachsen und die weiteren Schalen auf die jeweils äußerste Schale aufgewachsen werden.

Im Rahmen der vorliegenden Erfindung bezieht sich der Begriff "Passivierung" auf die Erzeugung einer Schutzschicht, welche die ZnSe-Partikeloberfläche gegen Umwelteinflüsse schützt und damit zu einer verbesserten Stabilität führt.

Im Rahmen der vorliegenden Erfindung bedeutet der Begriff "cadmiumfrei" im Zusammenhang mit den erfindungsgemäßen kolloidalen Quantenpunkten, dass keine Cadmium-Verbindungen zur Synthese der besagten Quantenpunkte eingesetzt werden.

Im Rahmen der vorliegenden Erfindung wird der Begriff "Ein-Topf-Verfahren" in seiner üblichen, dem Fachmann geläufigen Bedeutung verstanden und bezieht sich auf ein Syntheseverfahren, in welchem zwischen den einzelnen Syntheseschritten keine Reinigungsprozesse durchgeführt werden.

Die erfindungsgemäßen kolloidalen Quantenpunkte werden für den Einsatz in OLEDs, hybriden QD-Solarzellen, als stabile Fluoreszenzmarker in der Bioanalytik, in Fluoreszenzkollektoren, als lumineszierendes Sicherheitsmerkmal, in Röntgen-Szintillatoren, in organischen Feldeffekttransistoren und in der Beleuchtungstechnik verwendet.

In einer beispielhaften Ausführungsform der vorliegenden Erfindung liegt die Emissionswellenlänge der besagten kolloidalen Quantenpunkte zwischen 400 und 460 nm, bevorzugt zwischen 400 und 450 nm, mehr bevorzugt zwischen 410 und 440 nm.

In einer beispielhaften Ausführungsform der vorliegenden Erfindung liegt die Partikelgröße der besagten kolloidalen Quantenpunkte zwischen 2 und 10 nm, bevorzugt zwischen 2.3 und 9 nm, weiter bevorzugt zwischen 5 und 8 nm.

In einer beispielhaften Ausführungsform der vorliegenden Erfindung beträgt
a) für die Größenverteilung der kolloidalen Quantenpunkte die Standardabweichung nicht mehr als 20%, bevorzugt nicht mehr als 15%, weiter bevorzugt nicht mehr als 10% und/oder
b) die Halbwertsbreite (FWHM) des Emissionspeaks 10 bis 30 nm, bevorzugt 15 bis 20 nm.

Die kolloidalen Quantenpunkte gemäß der vorliegenden Erfindung umfassen
a) einen Kern umfassend Zinkselenid
b) mindestens eine auf den Kern aufgetragene Schale aus einem passivierenden Material,
wobei die kolloidalen Quantenpunkte cadmiumfrei sind.

Optional können auf die Zinkselenid-umfassenden Kerne der erfindungsgemäßen kolloidalen Quantenpunkte eine oder mehrere Schalen umfassend Zinkselenid aufgewachsen werden.

In einer bevorzugten Ausführungsform der vorliegenden Erfindung bestehen die kolloidalen Quantenpunkte aus
a) einem Kern umfassend Zinkselenid
b) einer oder mehrerer auf den Kern aufgewachsenen Schalen umfassend Zinkselenid
c) mindestens einer auf den Kern und/oder die letzte Schale aufgetragenen Schale aus einem passivierenden Material,
wobei die kolloidalen Quantenpunkte cadmiumfrei sind.

Bevorzugt können als passivierendes Material alle Verbindungen verwendet werden, welche die ZnSe-Partikeloberfläche vor Umwelteinflüssen schützen.

Weiter bevorzugt können als passivierendes Material Verbindungen eingesetzt werden ausgewählt aus der Gruppe umfassend ZnO, ZnS, MgO, MgS, MgSe, MgTe, CaO, CaS, CaSe, CaTe, AlN, GaN, GaP, GaAs, GaSb, SiC, TiN, BN, SiO₂, TiO₂, ZrO₂, HfO₂, MoO₃, WO₃, NiO, Al₂O₃, In₂O₃, und Gemische von diesen.

Besonders bevorzugt wird als passivierendes Material Zinksulfid (ZnS) eingesetzt.

Die ZnS-Schale passiviert Defekte an der ZnSe-Partikeloberfläche, was zu einer Verbesserung der Quantenausbeute und damit zu höheren Deviceeffizienzien führt. Weiterhin werden spektrale Verunreinigungen, die durch Defektzustände hervorgerufen werden, durch die Passivierung eliminiert, was die Farbsättigung erhöht.

Die erfindungsgemäßen kolloidalen Quantenpunkte enthalten keine toxischen Schwermetalle und unterliegen damit keinen regulatorischen Einschränkungen. Bisherige Ausführungen von blau emittierenden Quantenpunkt-LEDs verwenden cancerogene Cadmium-basierte Quantenpunkte, die aufgrund der Umweltgesetzgebung für die Anwendung nicht relevant sind.

Die Emissionswellenlänge der erfindungsgemäßen kolloidalen Quantenpunkte kann über die Größe des ZnSe-Kerns zwischen 400 und 460 nm eingestellt werden. Vorzugsweise liegt die Emissionswellenlänge der erfindungsgemäßen kolloidalen Quantenpunkte zwischen 400 und 460 nm, mehr bevorzugt zwischen 400 und 450 nm, weiter bevorzugt zwischen 410 und 440 nm.

Die Partikelgröße wird hierbei über die Stöchiometrie der Precursoren in der Nukleations- und der Wachstumsphase und/oder die Anzahl der ZnSe-Schalen eingestellt. Vorzugsweise liegt die Partikelgröße der erfindungsgemäßen kolloidalen Quantenpunkte zwischen 2 und 10 nm, bevorzugt zwischen 2.3 und 9 nm, weiter bevorzugt zwischen 5 und 8 nm. Die Bestimmung der Partikelgröße erfolgt bevorzugt mittels dem Fachmann geläufigen Elektronenmikroskopie-Methoden.

Im Rahmen der vorliegenden Erfindung bezieht sich der Begriff "Nukleationsphase" auf die Bildung der ZnSe-Kerne und der Begriff "Wachtstumsphase" auf den Wachtstumsprozess der auf den Kern aufgebrachten ZnSe-Schalen.

Bevorzugt kann die Größe der ZnSe-Kerne der erfindungsgemäßen kolloidalen Quantenpunkte nach der Nukleationsphase in einem Bereich von 1.5 bis 2 nm liegen. Nach dem optionalen Aufbringen einer oder mehrerer ZnSe-Schalen liegt die Partikelgröße bevorzugt in einem Bereich von 2.4 bis 6 nm.

Zur Einstellung des Wellenlängenbereiches liegt die Schichtdicke der ersten und jeder weiteren optionalen ZnSe-Schale bevorzugt in einem Bereich von 0.4 bis 3.6 nm, weiter bevorzugt in einem Bereich von 1.2 bis 3 nm.

Die Schichtdicke der Schale aus einem passivierenden Material liegt bevorzugt in einem Bereich von 0.3 bis 3 nm, weiter bevorzugt in einem Bereich von 2 bis 3 nm.

Die erfindungsgemäßen kolloidalen Quantenpunkte werden in exzellenten Größenverteilungen erhalten, wodurch der Peak der Bandlückenemission extrem schmal ist. Zusammen mit der Kontrollierbarkeit der Lage des Emissionspeaks erlaubt dies eine perfekte Einstellbarkeit der CIE-Farbkoordinaten des erzeugten blauen Lichtes Bevorzugt beträgt die für die Größenverteilung der erfindungsgemäßen kolloidalen Quantenpunkte die Standardabweichung nicht mehr als 20%, weiter bevorzugt nicht mehr als 15%, besonders bevorzugt nicht mehr als 10%. Bevorzugt liegt die Halbwertsbreite (FWHM) des Emissionspeaks der erfindungsgemäßen kolloidalen Quantenpunkte im Bereich von 10 bis 30 nm, besonders bevorzugt im Bereich von 15 bis 20 nm.

Vorzugsweise sind die erfindungsgemäßen kolloidalen Quantenpunkte mit organischen Oberflächenliganden beschichtet.

Die Verwendung von organischen Oberflächenliganden erlaubt die Dispergierbarkeit in unpolaren Lösungsmitteln, was die Verarbeitung der Quantenpunkte mittels kostengünstigen und flexiblen Lösungsprozessen wie Spin-Coating, Dip-Coating, Inkjet-Printing, Gravure Printing und ähnlichen erlaubt. Gleichzeitig wird der Organik-Anteil durch die Verwendung geeigneter Liganden so optimiert, dass in der dünnen Schicht eine hohe Dichte der Quantenpunkte erzielt wird und so eine effiziente Funktion im Device ermöglicht wird.

Bevorzugt können zur Beschichtung der erfindungsgemäßen kolloidalen Quantenpunkte alle organischen Oberflächenliganden verwendet werden, die eine Dispergierbarkeit der kolloidalen Quantenpunkte in unpolaren Lösungsmitteln ermöglichen.

Weiter bevorzugt können Liganden verwendet werden ausgewählt aus der Gruppe umfassend TOP (Triocylphosphan), TOPO (Trioctylphosphanoxid), Zinkcarboxylate, Carbonsäuren, Amine, Phosphonsäuren und Gemischen von diesen.

Bevorzugt weisen die ZnSe-Kerne der erfindungsgemäßen kolloidalen Quantenpunkte einen ZnSe-Anteil im Bereich von 40 bis 80 Gew.-% auf, weiter bevorzugt von 50 bis 80 Gew.-%.

Das Verfahren zur Herstellung der kolloidalen Quantenpunkte umfasst in einem Schritt a) das Bereitstellen einer Rohlösung, welche mindestens ein Zinkcarboxylat umfasst.

Die Rohlösung, welche mindestens ein Zinkcarboxylat umfasst, wird vorzugsweise durch das Inkontaktbringen eines Zinksalzes mit mindestens einer Carbonsäure erhalten.

Bevorzugt können als Zinksalze alle Zinksalze verwendet werden, die eine ausreichende Reaktivität aufweisen, um das entsprechende Zinkcarboxylat zu erhalten.

Weiter bevorzugt werden Zinksalze verwendet ausgewählt aus der Gruppe umfassend Zinkacetat, Zinkfluorid, Zinkchlorid, Zinkbromid, Zinkiodid, Zinknitrat, Zinktriflat, Zinktosylat, Zinkmesylat, Zinkoxid, Zinksulfat, Zinkacetylacetonat, Zink(Toluol-3,4-dithiolat), Zink(p-Toluol-sulfonat), Zinkdiethyl-dithio-carbamat, Zinkdebenzyl-dithiocarbamat und Gemische von diesen.

Bevorzugt können als Carbonsäure alle Verbindungen verwendet werden, die eine ausreichende Reaktivität aufweisen, um das entsprechende Zinkcarboxylat zu erhalten.

Weiter bevorzugt werden Carbonsäuren verwendet ausgewählt aus der Gruppe umfassend Essigsäure, Propionsäure, Buttersäure, Valeriansäure, Capronsäure, Heptansäure, Caprylsäure, Caprinsäure, Undecansäure, Laurinsäure, Myristinsäure, Palmitinsäure, Stearinsäure, Behensäure, Acrylsäure, Methacrylsäure, But-2-ensäure, But-3-ensäure, Pent-2-ensäure, Pent-4-ensäure, Hex-2-ensäure, Hex-3-ensäure, Hex-4-ensäure, Hex-5-ensäure, Hept-6-ensäure, Oct-2-ensäure, Dec-2-ensäure, Undec-10-ensäure, Dodec-5-ensäure, Ölsäure, Gadoleinsäure, Erucasäure, Linolsäure, α-Linolensäure, Calendulasäure, Eicosadiensäure, Eicosatriensäure, Arachidonsäure, Stearidonsäure, Benzoesäure, para-Toluinsäure, ortho-Toluinsäure, meta-Toluinsäure, Hydrozimtsäure, Naphtensäure, Zimtsäure, para-Toluolsulfonsäure und Gemische von diesen.

Die aufgeführten Zinksalze und Carbonsäuren können in jeglicher Form kombiniert und verwendet werden. Besonders bevorzugt werden Zinkoxid als Zinksalz und Caprylsäure als Carbonsäure verwendet.

Um das Zinksalz in das entsprechende Zinkcarboxylat zu überführen, können übliche, dem Fachmann bekannte Verfahren verwendet werden.

Das Verfahren zur Herstellung der kolloidalen Quantenpunkte umfasst in einem Schritt b) das Bereitstellen mindestens einer Selenquelle.

Bevorzugt werden als Selenquelle Verbindungen verwendet ausgewählt aus der Gruppe umfassend Trioctylphosphanselenid, Tri(n-butyl)phosphanselenid, Tri(sec-butyl)phosphanselenid, Tri(tert-butyl)phosphanselenid, Trimethylphosphanselenid, Triphenylphosphanselenid, Diphenylphosphanselenid, Phenylphosphanselenid, Cyclohexylphosphanselenid, Octaselenol, Dodecaselenol, Selenophenol, in Octadecen gelöstes elementares Selen, in Octadecen gelöstes Selendioxid, Selenourea, Bis(trimethylsilyl)selenid und Gemische von diesen.

Besonders bevorzugt wird Diphenyphosphanselenid als Selenquelle verwendet.

Das Verfahren zur Herstellung der kolloidalen Quantenpunkte umfasst in einem Schritt c) das Bereitstellen mindestens einer Schwefelquelle.

Bevorzugt werden als Schwefelquelle Verbindungen verwendet ausgewählt aus der Gruppe umfassend elementarer Schwefel, Octanthiol, Dodecanthiol, Octadecanthiol, Tributylphosphansulfid, Cyclohexylisothiocyanat, α-Toluolthiol, Ethylentrithiocarbonat, Allylmercaptan, Bis(trimethylsilyl)sulfid, Trioctylphosphansulfid und Gemische von diesen.

Besonders bevorzugt wird als Schwefelquelle Trioctylphosphansulfid verwendet.

Das Verfahren zur Herstellung der kolloidalen Quantenpunkte umfasst in einem Schritt d) das Inkontaktbringen der Rohlösung, welche mindestens ein Zinkcarboxylat umfasst aus Schritt a) mit mindestens einer Selenquelle aus Schritt b).

Vorzugsweise wird das Zinkcarboxylat für die Reaktion in einem organischen Lösungsmittel suspendiert. Als Lösungsmittel können alle organischen Lösungsmittel verwendet werden. Insbesondere kann das organische Lösungsmittel ausgewählt sein aus Kohlenwasserstoffen mit einem Siedepunkt von mindestens 250 °C und weiter bevorzugt von mindestens 300 °C bei Normaldruck. Beispielsweise kann das organische Lösungsmittel ausgewählt sein aus Octadecen und Paraffinöl, vorzugsweise Octadecen.

Bevorzugt liegen in Schritt d) das Zinkcarboxylat und die Selenquelle in einem stöchiometrischen Verhältnis im Bereich von 2:1 bis 20:1 vor, besonders bevorzugt beträgt das stöchiometrische Verhältnis 10:1.

In einer bevorzugten Ausführungsform wird die Suspension von Zinkcarboxylat in einem organischen Lösungsmittel auf eine erhöhte Reaktionstemperatur im Bereich von 200 bis 320 °C erhitzt, weiter bevorzugt auf 270 bis 290 °C. Bei dieser Temperatur wird die Selenquelle hinzugefügt, wobei die Zinkquelle bevorzugt in einem starken Überschuss von bis zu 20 stöchiometrischen Äquivalenten vorliegt, weiter bevorzugt von bis zu 10 stöchiometrischen Äquivalenten. Das Reaktionsgemisch wird für eine gewisse Zeit von bis zu 60 Minuten bei der gewählten erhöhten Reaktionstemperatur belassen, vorzugsweise weniger als 10 Minuten.

Die erhaltenen ZnSe-Kernnanopartikel weisen eine sehr niedrige Emissionswellenlänge im Bereich von etwa 380 bis 410 nm auf. Um diese in den für die Anwendung relevanteren Bereich bis 460 nm zu verschieben, umfasst das Verfahren einen optionalen Schritt e), welcher mindestens ein weiteres Inkontaktbringen der aus Schritt d) erhaltenen Reaktionslösung mit mindestens einer weiteren Selenquelle aus Schritt b) umfasst.

Bevorzugt werden als weitere Selenquelle Verbindungen verwendet ausgewählt aus der Gruppe umfassend Trioctylphosphanselenid, Tri(n-butyl)phosphanselenid, Tri(sec-butyl)phosphanselenid, Tri(tert-butyl)phosphanselenid, Trimethylphosphanselenid, Triphenylphosphanselenid, Diphenylphosphanselenid, Phenylphosphanselenid, Cyclohexylphosphanselenid, Octaselenol, Dodecaselenol, Selenophenol, in Octadecen gelöstes elementares Selen, in Octadecen gelöstes Selendioxid, Selenourea, Bis(trimethylsilyl)selenid und Gemische von diesen.

Die weitere Selenquelle muss nicht identisch sein mit der im ersten Wachstumsschritt verwendeten Selenquelle. Bevorzugt ist die weitere Selenquelle nicht identisch mit der im ersten Wachstumsschritt verwendeten Selenquelle.

Besonders bevorzugt wird als weitere Selenquelle Trioctylphosphanselenid verwendet.

Vorzugsweise wird die weitere Selenquelle bei Raumtemperatur hinzugefügt.

In einer bevorzugten Ausführungsform wird das Reaktionsgemisch nach Hinzufügen der weiteren Selenquelle auf eine Reaktionstemperatur im Bereich von 200 bis 320 °C erhitzt, mehr bevorzugt von 270 bis 290 °C. Das Reaktionsgemisch wird für eine gewisse Zeit von bis zu 60 Minuten bei der gewählten erhöhten Reaktionstemperatur belassen, vorzugsweise 10 bis 30 Minuten.

Das Verfahren zur Herstellung der kolloidalen Quantenpunkte umfasst in einem Schritt f) das Inkontaktbringen der aus Schritt d) und/oder Schritt e) erhaltenen Reaktionslösung mit mindestens einer Schwefelquelle aus Schritt c).

In einer bevorzugten Ausführungsform wird das Reaktionsgemisch nach Hinzufügen der Schwefelquelle auf eine Reaktionstemperatur im Bereich von 200 bis 320 °C, weiter bevorzugt von 270 bis 290 °C erhitzt. Das Reaktionsgemisch wird für eine gewisse Zeit von bis zu 60 Minuten bei der gewählten erhöhten Reaktionstemperatur belassen, vorzugsweise 10 bis 30 Minuten.

Schritt f) kann wiederholt werden, um eine dickere Passivierungsschicht und damit bessere Stabilität gegen Umwelteinflüsse zu erreichen

Nach Durchführung dieser Syntheseschritte werden die erfindungsgemäßen kolloidalen Quantenpunkte vom Lösungsmittel, von Nebenprodukten und von nicht abreagierten Precursoren durch mehrfaches Waschen mit einem leichtflüchtigen polaren Lösungsmittel abgetrennt, beispielsweise Butanon, Aceton, Isopropanol, Ethanol, Methanol, Acetonitril oder Gemischen davon.

Das dargestellte Verfahren bietet insbesondere den Vorteil, dass die erfindungsgemäßen kolloidalen Quantenpunkte in einem Ein-Topf-Verfahren hergestellt werden können. Dies ermöglicht ein Verfahren ohne Reinigungsprozesse zwischen den einzelnen Schritten, woraus sich ein Kostenvorteil gegenüber anderen Verfahren ergibt.

Die erfindungsgemäßen kolloidalen Quantenpunkte können aufgrund ihrer vorteilhaften Eigenschaften insbesondere in OLEDs, hybriden QD-organischen Solarzellen, als stabile Fluoreszenzmarker in der Bioanalytik, in Fluoreszenzkollektoren durch Einbettung in transparente organische Matrices, in der Sicherheitstechnologie als lumineszierendes Sicherheitsmerkmal, in neuartigen Röntgenstrahl-Szintillatoren, in organischen Feldeffekttransistoren und in der Beleuchtungstechnik eingesetzt werden.

Optoelektronische Bauelemente, in denen die erfindungsgemäßen kolloidalen Quantenpunkte eingesetzt werden, zeigen insbesondere eine günstige schmalbandige Elektrolumineszenz.

Das Verfahren zur Herstellung eines optoelektronischen Bauelements umfasst in einem Schritt a) das Bereitstellen einer transparenten Anode.

Bevorzugt umfasst die besagte Anode Indiumzinnoxid.

Das Verfahren zur Herstellung eines optoelektronischen Bauelements umfasst in einem Schritt b) das Bereitstellen einer lochinjizierenden Schicht.

Bevorzugt umfasst die besagte lochinjizierende Schicht PEDOT:PSS (Poly-3,4-ethylendioxythiophen Polystyrolsulfonat).

Das Verfahren zur Herstellung eines optoelektronischen Bauelements umfasst in einem Schritt c) das Bereitstellen einer lochleitenden Schicht.

Bevorzugt werden als lochleitende Schicht Materialien verwendet ausgewählt aus der Gruppe umfassend Poly-TPD (Poly(*N,N*'-bis(4-butylphenyl)-*N,N*'-bis(phenyl)benzidin)), TAPC (1- Bis[4-[*N*, *N*'-di(*p-*tolyl)amino]phenyl]cyclohexan), CBP (4,4'-Bis(carbazol-9-yl)biphenyl), PVK (Poly(*N*-vinylcarbazol)), TCTA (4,4',4"-Tris(carbazol-9-yl)triphenylamin), P3HT (Poly(3-hexylthiophen-2,5-diyl)), MEH-PPV (Poly[2-methoxy-5-(2-ethylhexyloxy)-1,4-phenylenevinylen]), MoO₃ (Molybdäntrioxid), ZrO₂ (Zirkoniumdioxid), NiO (Nickeloxid), WO₃ (Wolframtrioxid) oder Gemischen daraus.

Besonders bevorzugt umfasst die lochleitende Schicht TCTA.

Das Verfahren zur Herstellung eines optoelektronischen Bauelements umfasst in einem Schritt d) das Bereitstellen einer Schicht umfassend die erfindungsgemäßen kolloidalen Quantenpunkte.

Das Verfahren zur Herstellung eines optoelektronischen Bauelements umfasst in einem Schritt e) das Bereitstellen einer elektronenleitenden Schicht.

Bevorzugt werden als elektronenleitende Schicht Materialien verwendet ausgewählt aus der Gruppe umfassend TPBI (1,3,5-Tris(1-phenyl-1*H*-benzimidazol-2-yl)benzol), ZnO (Zinkoxid), TiO₂ (Titandioxid), TBPO (2-(4-Biphenyl)-5-(4-*tert-*butylphenyl)-1,3,4-oxadiazol) oder Gemische daraus.

Besonders bevorzugt umfasst die elektronenleitende Schicht TPBI.

Das Verfahren zur Herstellung eines optoelektronischen Bauelements umfasst in einem optionalen Schritt f) das Bereitstellen einer elektroneninjizierenden Schicht.

Bevorzugt werden als elektroneninjizierende Schicht Materialien verwendet ausgewählt aus der Gruppe umfassend LiF, CsF, Cs₂CO₃ oder Gemischen daraus.

Das Verfahren zur Herstellung eines optoelektronischen Bauelements umfasst in einem Schritt g) das Bereitstellen einer Metallkathode.

Bevorzugt werden Metallkathoden verwendet, welche Materialien umfassen ausgewählt aus der Gruppe umfassend Mg, Ba, Ca, Al, Ag, Au oder Gemischen daraus.

Besonders bevorzugt werden Metallkathoden verwendet, welche Ca/Ag umfassen.

Das optoelektronische Bauelement wird in einem Schichtaufbau hergestellt, wobei die einzelnen Schichten jeweils die Materialen aus den Schritten a) bis g) umfassen.

Die Quantenpunktschicht wird durch durch Spin-Coating, Dip-Coating, Inkjet-Printing, Gravure Printing und/oder Spray-Coating auf die lochleitende Schicht aufgebracht.

Bevorzugt wird für die Quantenpunkte ein organisches Lösungsmittel verwendet, das die lochleitende Schicht nicht beschädigt.

Weiter bevorzugt werden als Lösungsmittel für die Quantenpunkte Lösungsmittel verwendet ausgewählt aus der Gruppe umfassend Alkane wie Hexan, Heptan, Octan, Nonan, Decan, Dodecan, Undecan, Cyclohexan, Decalin oder verzweigte oder cyclische Isomere davon, Aromaten wie Toluol, Tetralin, Benzol, Chlorbenzol, o-Dichlorbenzol, *m*-Dichlorbenzol, *p*-Dichlorbenzol, Xylen, Mesitylen, oder Gemische daraus.

Besonders bevorzugt wird als Lösungsmittel für die Quantenpunkte Nonan verwendet.

### Kurze Beschreibung der Figuren

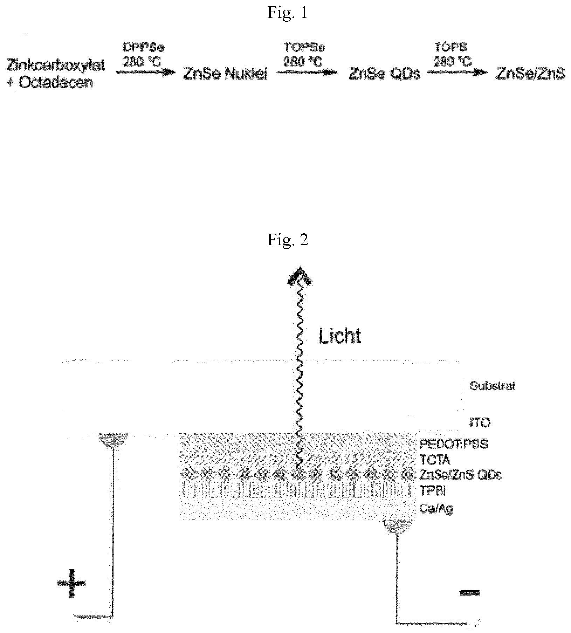

**Fig. 1** zeigt das Schema der Ein-Topf-Synthese von ZnSe/ZnS-Quantenpunkten mit einstellbarer Partikelgröße. Die Synthese umfasst die ZnSe-Kern-Nukleation, einen ZnSe-Kernwachstumsschritt und einen ZnS-Schalenwachstumsschritt.
**Fig. 2** zeigt das Device-Schema einer Quantenpunkt-LED mit einer Monolage von blau emittierenden Quantenpunkten und geeigneten Ladungsträger-Transportschichten sowie Elektroden.

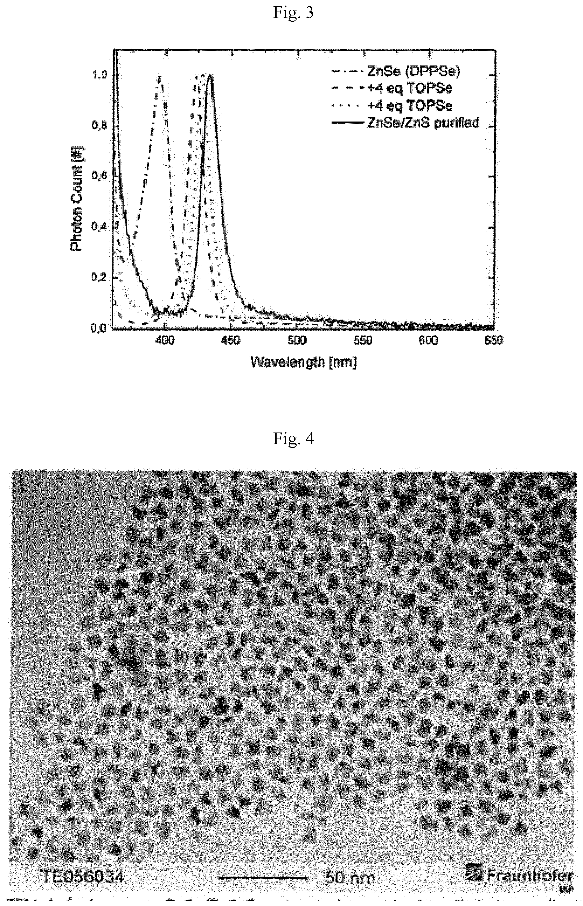

**Fig. 3** zeigt das Photolumineszenzspektrum der einzelnen Syntheseschritte umfassend die Kern-Nukleation, das Kernwachstum zur Verschiebung der Emissionswellenlänge auf 434 nm, sowie die Schalenabscheidung inklusive Aufarbeitung und Reiniung.
**Fig. 4** zeigt eine TEM-Aufnahme von ZnSe/ZnS-Quantenpunkten mit einer Emissionswellenlänge von 434 nm

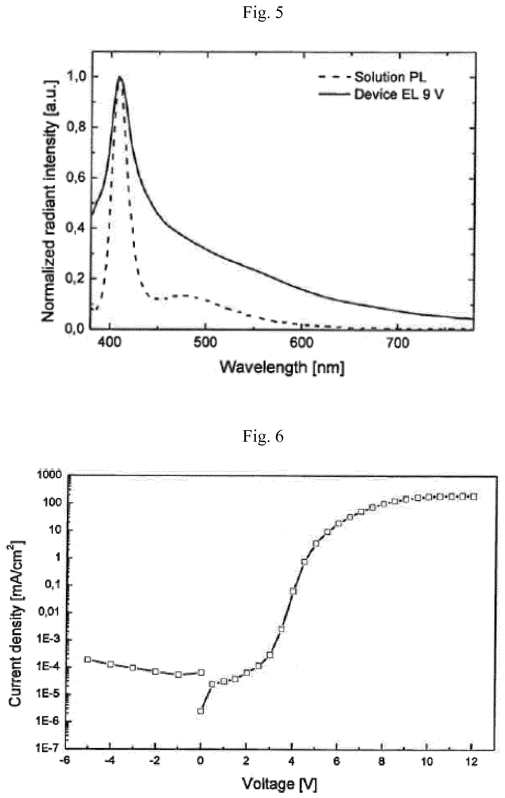

**Fig. 5** zeigt Photo- und Elektrolumineszenz-Spektren von ZnSe/ZnS-Quantenpunkten in Nonan-Lösung (PL) bzw. als dünne Schicht in einem Device.
**Fig. 6** zeigt die Stromdichte-Spannungskennlinie einer QLED mit ZnSe/ZnS-Quantenpunkten.

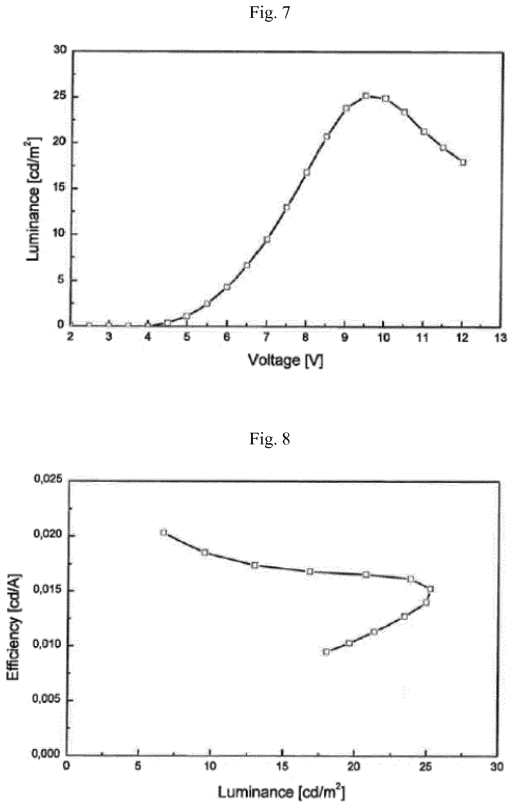

**Fig. 7** zeigt die Leuchtdichte-Spannungs-Kennlinie einer QLED mit ZnSe/ZnS-Quantenpunkten.
**Fig. 8** zeigt die Stromeffizienz-Leuchtdichte-Kennlinie einer QLED mit ZnSe/ZnS-Quantenpunkten.

### Ausführungsbeispiele

### Triocylphosphanselenid-Stammlösung

Eine Stammlösung von Triocylphosphanselenid in Triocylphosphan der Konzentration 1 M wurde durch das Auflösen von elementarem Selen (25 mmol, 2.0 g) in Triocylphosphan (25 mL) hergestellt. Die Mischung wurde für mehrere Stunden bei 60 °C gerührt, bis eine klare Lösung erhalten wurde.

### Diphenylphosphanselenid-Stammlösung

Eine Stammlösung von Diphenylphosphanselenid in Toluol der Konzentration 0.15 M wurde durch das Auflösen von elementarem Selen (53.7 mmol, 4.2 g) in Diphenylphosphan (53.7 mmol, 10 g) hergestellt. Die Mischung wurde unter Argonatmosphäre für mehrere Stunden bei 120 °C gerührt, bis eine klare orange Schmelze erhalten wurde. Diese wurde nach Abkühlung auf Raumtemperatur in 358 mL wasserfreiem Toluol gelöst.

### Beispiel Zinkcaprylat-Pulver

Zinkcaprylat wurde durch die Umsetzung von Zinkoxid (100 mmol, 8.1 g) mit Caprylsäure (200 mmol, 28.8 g) in einer Toluol-Suspension hergestellt. Die Suspension wurde unter Rückflussbedingungen für einige Stunden gerührt, bis eine klare Lösung erhalten wurde. Nach Entfernung des Lösungsmittels wurde das erhaltene Zinkcaprylat mehrere Stunden bei 50 °C unter Vakuum getrocknet.

### Beispiel ZnSe-Kernsynthese durch Heißinjektion

In einem Dreihalskolben wurde Zinkcaprylat (1 mmol, 0.35 g) in 1-Octadecen (2 mL) suspendiert und durch mehrfaches Evakuieren und Fluten mit Argon unter eine inerte Atmosphäre gesetzt. Die Mischung wurde unter Rühren auf 280 °C erhitzt, wobei eine klare Lösung erhalten wurde. Die dieser Temperatur wurde Diphenylphosphanselenid (0.1 mmol, 0.67 mL einer 0.15 M Stammlösung in Toluol) durch schnelle Injektion hinzugefügt. Das Toluol wurde durch einen Argon-Strom entfernt. Die Reaktionsmischung wurde für fünf Minuten bei 280 °C gehalten und anschließend auf Raumtemperatur abgekühlt. Die erhaltene schwach gelbe Lösung luminesziert bei 395 nm, FHWM 23 nm, QY 24%.

### ZnSe- Wachstum

Zu einer Rohlösung von ZnSe-Kernpartikeln wurde bei Raumtemperatur Trioctylphosphanselenid (0.4 mmol, 0.4 mL einer 1 M Stammlösung in Triocylphosphan) hinzugefügt. Das Gemisch wurde auf 280 °C erhitzt, dort für 20 Minuten gehalten, und anschließend auf Raumtemperatur abgekühlt. Die erhaltene Partikel-Lösung luminesziert bei 423 nm, FWHM 15 nm, QY 33%.

### ZnS-Schalensynthese

Zu einer Rohlösung von ZnSe-Nanopartikeln wurde im Argon-Gegenstrom Zinkcaprylat (1 mmol, 0.35 g) sowie Triocylphosphansulfid (1 mmol, 1 mL einer Stammlösung in Trioctylphosphan) hinzugefügt. Das Gemisch wurde auf 280 °C erhitzt, dort für 20 Minuten gehalten und anschließend auf Raumtemperatur abgekühlt. Die erhaltenen Kern-Schale-Partikel wurden mit Aceton ausgefällt und mehrfach gewaschen, um 80 g des gelben Nanopartikel-Pulvers zu erhalten. Laut thermogravimetrischer Analyse beträgt der Anteil der anorganischen Komponenten 76 wt.-%. Nach Redispersion in Nonan lumineszieren die Partikel bei einer Wellenlänge von 434 nm, FWHM 16 nm, QY 8%.

### Devicepräparation

Mit ITO (Indiumzinnoxid) beschichtete Glassubstrate wurden durch Spülen mit Acton, Isopropanol und Wasser gereinigt und im Stickstoffstrom getrocknet. PEDOT:SS (Poly-3,4-ethylendioxythiophen Polystyrolsulfonat, Heraeus) wurde durch Spin-Coating aus einem Methanol-Isopropanol-Wasser-Gemisch auf die ITO-Substrate aufgebracht und die Schicht anschließend bei 180 °C für 60 min getempert. Anschließend wurde TCTA (4,4',4"-Tris(carbazol-9-yl)triphenylamin, Sensient) aus einer Toluol-Lösung auf die PEDOT-Schicht aufgeschleudert. Die TCTA-Schicht wurde bei 130 °C für 30 min getrocknet. Die ZnSe/ZnS-Kern-Schale-Quantenpunkte wurden in Nonan dispergiert und ebenfalls durch Spin-Coating auf die TCTA-Schicht aufgebracht, worauf die ZnSe-Schicht für 30 min bei 100 °C getrocknet wurde. Anschließend wurden nacheinander dünne Schichten von TPBI (1,3,5-Tris(1-phenyl-1H-benzimidazol-2-yl)benzol, LumTec), Calcium und Silber durch thermische Verdampfung aufgebracht. Nach Verkapselung der Proben wurden diese elektrooptisch charakterisiert. Alle Prozessschritte wurden in einem Glovebox-System unter Inertatmosphäre ausgeführt.

## Patentansprüche

1. Verfahren zur Herstellung von kolloidalen Quantenpunkten umfassend
i) einen Kern umfassend Zinkselenid
ii) optional eine oder mehrere auf den Kern aufgewachsene Schalen umfassend Zinkselenid
iii) mindestens eine auf den Kern und/oder die letzte Schale aufgetragene Schale aus einem passivierenden Material,
wobei die kolloidalen Quantenpunkte cadmiumfrei sind,
umfassend:
a) Bereitstellen einer Rohlösung, welche mindestens ein Zinkcarboxylat und ein organisches Lösungsmittel ausgewählt aus Kohlenwasserstoffen mit einem Siedepunkt von mindestens 250 °C bei Normaldruck umfasst,
b) Bereitstellen mindestens einer Selenquelle,
c) Bereitstellen mindestens einer Schwefelquelle,
d) Inkontaktbringen der Rohlösung, welche mindestens ein Zinkcarboxylat umfasst aus Schritt a) mit mindestens einer Selenquelle aus Schritt b),
e) optional mindestens ein weiteres Inkontaktbringen der aus Schritt d) erhaltenen Reaktionslösung mit mindestens einer weiteren Selenquelle aus Schritt b), und
f) Inkontaktbringen der aus Schritt d) und/oder Schritt e) erhaltenen Reaktionslösung mit mindestens einer Schwefelquelle aus Schritt c),
wobei die kolloidalen Quantenpunkte in einem Ein-Topf-Verfahren hergestellt werden und die Partikelgröße in den Schritten d) und e) über das stöchiometrische Verhältnis der Rohlösung, welche mindestens ein Zinkcarboxylat umfasst aus Schritt a) und der Selenquelle aus Schritt b) und/oder über die Anzahl der Wachstumsschritte gemäß Schritt e) bestimmt wird.

2. Verfahren nach Anspruch 1, wobei
(i) die Emissionswellenlänge der besagten Quantenpunkte zwischen 400 und 460 nm liegt und/oder
(ii) die besagten Quantenpunkte eine Partikelgröße zwischen 2 und 10 nm aufweisen.

3. Verfahren nach Anspruch 1 oder 2, wobei die Halbwertsbreite (FWHM) des Emissionspeaks der kolloidalen Quantenpunkte in einem Bereich von 10 bis 30 nm liegt.

4. Verfahren nach Anspruch 1, wobei die Rohlösung, welche mindestens ein Zinkcarboxylat umfasst, erhalten wird durch Inkontaktbringen eines Zinksalzes, ausgewählt aus der Gruppe umfassend Zinkacetat, Zinkfluorid, Zinkchlorid, Zinkbromid, Zinkiodid, Zinknitrat, Zinktriflat, Zinktosylat, Zinkmesylat, Zinkoxid, Zinksulfat, Zinkacetylacetonat, Zink(Toluol-3,4-dithiolat), Zink(p-Toluol-sulfonat), Zinkdiethyl-dithio-carbamat, Zinkdebenzyl-dithiocarbamat und Gemische von diesen, mit mindestens einer Carbonsäure, ausgewählt aus der Gruppe umfassend Essigsäure, Propionsäure, Buttersäure, Valeriansäure, Capronsäure, Heptansäure, Caprylsäure, Caprinsäure, Undecansäure, Laurinsäure, Myristinsäure, Palmitinsäure, Stearinsäure, Behensäure, Acrylsäure, Methacrylsäure, But-2-ensäure, But-3-ensäure, Pent-2-ensäure, Pent-4-ensäure, Hex-2-ensäure, Hex-3-ensäure, Hex-4-ensäure, Hex-5-ensäure, Hept-6-ensäure, Oct-2-ensäure, Dec-2-ensäure, Undec-10-ensäure, Dodec-5-ensäure, Ölsäure, Gadoleinsäure, Erucasäure, Linolsäure, α-Linolensäure, Calendulasäure, Eicosadiensäure, Eicosatriensäure, Arachidonsäure, Stearidonsäure, Benzoesäure, para-Toluinsäure, ortho-Toluinsäure, meta-Toluinsäure, Hydrozimtsäure, Naphtensäure, Zimtsäure, para-Toluolsulfonsäure und Gemische von diesen.

5. Verfahren nach Anspruch 1, wobei die Selenquelle ausgewählt ist aus der Gruppe umfassend Trioctylphosphanselenid, Tri(n-butyl)phosphanselenid, Tri(sec-butyl)phosphanselenid, Tri(tert-butyl)phosphanselenid, Trimethylphosphanselenid, Triphenylphosphanselenid, Diphenylphosphanselenid, Phenylphosphanselenid, Cyclohexylphosphanselenid, Octaselenol, Dodecaselenol, Selenophenol, in Octadecen gelöstes elementares Selen, in Octadecen gelöstes Selendioxid, Selenourea, Bis(trimethylsilyl)selenid und Gemische von diesen.

6. Verfahren nach Anspruch 1, wobei die Schwefelquelle ausgewählt ist aus der Gruppe umfassend elementarer Schwefel, Octanthiol, Dodecanthiol, Octadecanthiol, Tributylphosphansulfid, Cyclohexylisothiocyanat, α-Toluolthiol, Ethylentrithiocarbonat, Allylmercaptan, Bis(trimethylsilyl)sulfid, Trioctylphosphansulfid und Gemische von diesen.

7. Verfahren nach Anspruch 1, wobei
(i) die Rohlösung, welche mindestens ein Zinkcarboxylat umfasst aus Schritt a) vor dem Inkontaktbringen mit der Selenquelle aus Schritt b) in Schritt d) auf eine Temperatur ein einem Bereich von 200 °C bis 320 °C, bevorzugt in einem Bereich von 270 °C bis 290 °C erhitzt wird,
(ii) optional die aus Schritt d) erhaltene Reaktionslösung einer weiteren Selenquelle aus Schritt b) in Kontakt gebracht wird und nach dem Inkontaktbringen auf eine Temperatur ein einem Bereich von 200 °C bis 320 °C, bevorzugt in einem Bereich von 270 °C bis 290 °C erhitzt wird, und
(iii) die aus Schritt d) und/oder Schritt e) erhaltene Reaktionslösung nach dem Inkontaktbringen mit der Schwefelquelle aus Schritt c) auf eine Temperatur ein einem Bereich von 200 °C bis 320 °C, bevorzugt in einem Bereich von 270 °C bis 290 °C erhitzt wird.

## Claims

1. Process for producing colloidal quantum dots comprising
i) a core comprising zinc selenide
ii) optionally one or more shells grown onto the core and comprising zinc selenide
iii) at least one shell of a passivating material, applied to the core and/or to the last shell,
the colloidal quantum dots being cadmium-free, which comprises:
a) providing a raw solution which comprises at least one zinc carboxylate and an organic solvent selected from hydrocarbons having a boiling point of at least 250°C at standard pressure,
b) providing at least one selenium source,
c) providing at least one sulfur source,
d) contacting the raw solution comprising at least one zinc carboxylate from step a) with at least one selenium source from step b),
e) optionally contacting the reaction solution obtained from step d) at least one further time, with at least one further selenium source from step b), and
f) contacting the reaction solution obtained from step d) and/or step e) with at least one sulfur source from step c),
wherein the colloidal quantum dots are produced in a one-pot process and the particle size in steps d) and e) is defined via the stoichiometric ratio of the raw solution comprising at least one zinc carboxylate from step a) and the selenium source from step b) and/or via the number of growth steps in step e).

2. Process according to Claim 1, wherein
(i) the emission wavelength of said quantum dots is between 400 and 460 nm and/or
(ii) said quantum dots have a particle size between 2 and 10 nm.

3. Process according to Claim 1 or 2, wherein the full width at half maximum (FWHM) of the emission peak of the colloidal quantum dots is within a range from 10 to 30 nm.

4. Process according to Claim 1, wherein the raw solution comprising at least one zinc carboxylate is obtained by contacting a zinc salt, selected from the group encompassing zinc acetate, zinc fluoride, zinc chloride, zinc bromide, zinc iodide, zinc nitrate, zinc triflate, zinc tosylate, zinc mesylate, zinc oxide, zinc sulfate, zinc acetylacetonate, zinc toluene-3,4-dithiolate, zinc(p-toluenesulfonate), zinc diethyldithiocarbama te, zinc deibenzyldithiocarbamate and mixtures of these, with at least one carboxylic acid selected from the group encompassing acetic acid, propionic acid, butyric acid, valeric acid, caproic acid, heptanoic acid, caprylic acid, capric acid, undecanoic acid, lauric acid, myristic acid, palmitic acid, stearic acid, behenic acid, acrylic acid, methacrylic acid, but-2-enoic acid, but-3-enoic acid, pent-2-enoic acid, pent-4-enoic acid, hex-2-enoic acid, hex-3-enoic acid, hex-4-enoic acid, hex-5-enoic acid, hept-6-enoic acid, oct-2-enoic acid, dec-2-enoic acid, undec-10-enoic acid, dodec-5-enoic acid, oleic acid, gadoleic acid, erucic acid, linoleic acid, α-linolenic acid, calendic acid, eicosadienoic acid, eicosatrienoic acid, arachidonic acid, stearidonic acid, benzoic acid, para-toluic acid, ortho-toluic acid, meta-toluic acid, hydrocinnamic acid, naphthenic acid, cinnamic acid, para-toluenesulfonic acid and mixtures of these.

5. Process according to Claim 1, wherein the selenium source is selected from the group encompassing trioctylphosphane selenide, tri(n-butyl)phosphane selenide, tri(sec-butyl)phosphane selenide, tri(tert-butyl)phosphane selenide, trimethylphosphane selenide, triphenylphosphane selenide, diphenylphosphane selenide, phenylphosphane selenide, cyclohexylphosphane selenide, octaselenol, dodecaselenol, selenophenol, elemental selenium dissolved in octadecene, selenium dioxide dissolved in octadecene, selenourea, bis(trimethylsilyl) selenide and mixtures of these.

6. Process according to Claim 1, wherein the sulfur source is selected from the group encompassing elemental sulfur, octanethiol, dodecanethiol, octadecanethiol, tributylphosphane sulfide, cyclohexyl isothiocyanate, α-toluenethiol, ethylene trithiocarbonate, allyl mercaptan, bis(trimethylsilyl) sulfide, trioctylphosphane sulfide and mixtures of these.

7. Process according to Claim 1, wherein
(i) the raw solution comprising at least one zinc carboxylate from step a), before being contacted with the selenium source from step b), is heated in step d) to a temperature within a range from 200°C to 320°C, preferably within a range from 270°C to 290°C,
(ii) optionally the reaction solution obtained from step d) is contacted with a further selenium source from step b) and, after contacting, is heated to a temperature within a range from 200°C to 320°C, preferably within a range from 270°C to 290°C, and
(iii) the reaction solution obtained from step d) and/or step e), after being contacted with the sulfur source from step c), is heated to a temperature within a range from 200°C to 320°C, preferably within a range from 270°C to 290°C.

## Revendications

1. Procédé de fabrication de points quantiques colloïdaux, comprenant :
i) un noyau comprenant du séléniure de zinc,
ii) éventuellement une ou plusieurs enveloppes comprenant du séléniure de zinc formées sur le noyau,
iii) au moins une enveloppe en un matériau passivant appliquée sur le noyau et/ou la dernière enveloppe,
les points quantiques colloïdaux étant exempts de cadmium,
comprenant :
a) la préparation d'une solution brute, qui comprend au moins un carboxylate de zinc et un solvant organique choisi parmi les hydrocarbures ayant un point d'ébullition d'au moins 250 °C à pression normale,
b) la préparation d'au moins une source de sélénium,
c) la préparation d'au moins une source de soufre,
d) la mise en contact de la solution brute qui comprend au moins un carboxylate de zinc de l'étape a) avec au moins une source de sélénium de l'étape b),
e) éventuellement au moins une mise en contact supplémentaire de la solution réactionnelle obtenue à l'étape d) avec au moins une source de sélénium supplémentaire de l'étape b), et
f) la mise en contact de la solution réactionnelle obtenue à l'étape d) et/ou à l'étape e) avec au moins une source de soufre de l'étape c),
les points quantiques colloïdaux étant fabriqués par un procédé monotope, et la taille de particule aux étapes d) et e) étant déterminée par le rapport stœchiométrique entre la solution brute, qui comprend au moins un carboxylate de zinc, de l'étape a) et la source de sélénium de l'étape b), et/ou par le nombre d'étapes de croissance selon l'étape e).

2. Procédé selon la revendication 1, dans lequel
(i) la longueur d'onde d'émission desdits points quantiques est comprise entre 400 et 460 nm, et/ou
(ii) lesdits points quantiques présentent une taille de particule comprise entre 2 et 10 nm.

3. Procédé selon la revendication 1 ou 2, dans lequel la largeur à mi-hauteur (FWHM) du pic d'émission des points quantiques colloïdaux se situe dans une plage allant de 10 à 30 nm.

4. Procédé selon la revendication 1, dans lequel la solution brute, qui comprend au moins un carboxylate de zinc, est obtenue par mise en contact d'un sel de zinc, choisi dans le groupe comprenant l'acétate de zinc, le fluorure de zinc, le chlorure de zinc, le bromure de zinc, l'iodure de zinc, le nitrate de zinc, le triflate de zinc, le tosylate de zinc, le mésylate de zinc, l'oxyde de zinc, le sulfate de zinc, l'acétylacétonate de zinc, le toluène-3,4-dithiolate de zinc, le p-toluène-sulfonate de zinc, le diéthyl-dithio-carbamate de zinc, le dibenzyl-dithiocarbamate de zinc et des mélanges de ceux-ci, avec au moins un acide carboxylique, choisi dans le groupe comprenant l'acide acétique, l'acide propionique, l'acide butyrique, l'acide valérique, l'acide caproïque, l'acide heptanoïque, l'acide caprylique, l'acide caprique, l'acide undécanoïque, l'acide laurique, l'acide myristique, l'acide palmitique, l'acide stéarique, l'acide béhénique, l'acide acrylique, l'acide méthacrylique, l'acide but-2-énoïque, l'acide but-3-énoïque, l'acide pent-2-énoïque, l'acide pent-4-énoïque, l'acide hex-2-énoïque, l'acide hex-3-énoïque, l'acide hex-4-énoïque, l'acide hex-5-énoïque, l'acide hept-6-énoïque, l'acide oct-2-énoïque, l'acide déc-2-énoïque, l'acide undéc-10-énoïque, l'acide dodéc-5-énoïque, l'acide oléique, l'acide gadoléique, l'acide érucique, l'acide linoléique, l'acide α-linolénique, l'acide calendulique, l'acide eicosadiénoïque, l'acide eicosatriénoïque, l'acide arachidonique, l'acide stéaridonique, l'acide benzoïque, l'acide para-toluinique, l'acide ortho-toluinique, l'acide méta-toluinique, l'acide hydrocinnamique, l'acide naphténique, l'acide cinnamique, l'acide para-toluène-sulfonique et les mélanges de ceux-ci.

5. Procédé selon la revendication 1, dans lequel la source de sélénium est choisie dans le groupe comprenant le séléniure de trioctylphosphane, le séléniure de tri(n-butyl)phosphane, le séléniure de tri(sec-butyl)phosphane, le séléniure de tri(tert-butyl)phosphane, le séléniure de triméthylphosphane, le séléniure de triphénylphosphane, le séléniure de diphénylphosphane, le séléniure de phénylphosphane, le séléniure de cyclohexylphosphane, l'octasélénol, le dodécasélénol, le sélénophénol, le sélénium élémentaire dissous dans de l'octadécène, le dioxyde de sélénium dissous dans de l'octadécène, la sélénourée, le séléniure de bis(triméthylsilyle) et les mélanges de ceux-ci.

6. Procédé selon la revendication 1, dans lequel la source de soufre est choisie dans le groupe comprenant le soufre élémentaire, l'octane-thiol, le dodécane-thiol, l'octadécane-thiol, le sulfure de tributylphosphane, l'isothiocyanate de cyclohexyle, l'α-toluène-thiol, le trithiocarbonate d'éthylène, l'allylmercaptan, le sulfure de bis(triméthylsilyle), le sulfure de trioctylphosphane et les mélanges de ceux-ci.

7. Procédé selon la revendication 1, dans lequel
(i) la solution brute, qui comprend au moins un carboxylate de zinc, de l'étape a) est portée à une température dans une plage allant de 200 °C à 320 °C, de préférence dans une plage allant de 270 °C à 290 °C, à l'étape d) avant la mise en contact avec la source de sélénium de l'étape b),
ii) éventuellement, la solution réactionnelle obtenue à l'étape d) est mise en contact avec une source de sélénium supplémentaire de l'étape b) et, après la mise en contact, portée à une température dans une plage allant de 200 °C à 320 °C, de préférence dans une plage allant de 270 °C à 290 °C, et
(iii) la solution réactionnelle obtenue à l'étape d) et/ou à l'étape e) est portée à une température dans une plage allant de 200 °C à 320 °C, de préférence dans une plage allant de 270 °C à 290 °C, après la mise en contact avec la source de soufre de l'étape c).
